# EUROPEAN PATENT SPECIFICATION

(11) **EP 4 192 566 B1**
(45) Date of publication and mention of the grant of the patent: **03.06.2026**
(21) Application number: 20761405.8
(22) Date of filing: 07.08.2020
(51) Int. Cl.: A61M 25/10, B29C 63/10, B29C 63/24

(54) **COMPOSITE MEDICAL BALLOON WITH HYBRID OUTER LAYER AND RELATED PRODUCTION METHOD**
MEDIZINISCHER VERBUNDBALLON MIT HYBRID-AUSSENSCHICHT UND VERFAHREN ZUR DESSEN HERSTELLUNG
BALLON MÉDICAL COMPOSITE AVEC COUCHE EXTÉRIEURE HYBRIDE ET PROCÉDÉ DE FABRICATION CORRESPONDENT

(43) Date of publication of application: 14.06.2023
(73) Proprietor: C. R. Bard, Inc., Franklin Lakes, NJ 07417 (US)
(72) Inventor: THOMAS, Kyle Andrew, Phoenix, AZ 85044 (US)
(74) Representative: Hoffmann Eitle
(86) International application number: PCT/US2020/045433
(87) International publication number: WO 2022/031297

(56) References cited:
- WO-A1-2014/176422
- US-A1- 2007 138 694
- US-A1- 2010 243 135

## Description

### BACKGROUND

Medical balloons are widely used in medical procedures. Typically, an uninflated medical balloon is inserted into a body space. When the medical balloon is inflated, the volume of the medical balloon expands, and the body space is similarly expanded.

In procedures such as angioplasty, the medical balloon may be used to open a collapsed or blocked artery. A typical medical balloon includes a central barrel portion between tapered or conical portions. The medical balloon may be provided in a non-compliant form by including one or more fiber layers, but compliant and semi-compliant forms are also known in the art.

To provide an outer layer for a medical balloon, past proposals have been made for cutting the ends of a tubular film longitudinally to form radially divided segments that may overlap to surround the tapered end more precisely when attached thereto. However, this leads to the creation of a number of longitudinal seams, which may be undesirable for some applications. Furthermore, if not done with great care, the results from forming the seams can be inconsistent, can lead to longitudinally extending fins. The manufacturing process involving the cutting of an outer layer preform can be complicated and costly, typically requiring an entirely manual process.

US 2007/138694 A1 relates to an expandable medical device with a tubular body formed of a wrapped sheet of porous polymeric material fused together to form an angled fused seam. US 2010/243135 A1 relates to a non-compliant fiber-reinforced medical balloon comprising a base layer formed by winding a film or tape over the surface of the balloon.

Accordingly, a need is identified for a medical balloon that overcomes any or all of the foregoing limitations and possibly others that have yet to be identified.

### SUMMARY OF THE INVENTION

The present invention is defined in claim 1 while preferred embodiments are set forth in the dependent claims.

The object of the invention is to provide a composite medical balloon including a hybrid layer including a tube for covering a barrel portion and a spiral wrapping for covering one or both of the tapered or cone portions.

According to the invention, a composite medical balloon comprises a base balloon layer including first and second tapered portions with a barrel portion therebetween. A hybrid layer includes a tube covering the barrel portion and a spiral wrapping covering at least one of the first and second tapered portions.

In one embodiment, the tube comprises an extruded or blow-molded material, which is substantially equal in length to the barrel portion of the base balloon layer. The tube may comprise a polyamide, and the base balloon layer may also comprise a polyamide. The spiral wrapping may comprise a spirally wound ribbon of material extending from a proximal end of the at least one tapered portion to a distal end of the at least one tapered portion. The spiral wrapping may overlap with the tube at a transition from the at least one tapered portion to the barrel portion. The spiral wrapping may comprise a polyether block amide, such as PEBAX, and may cover both tapered portions of the base balloon layer. The balloon may further include a fiber layer over the base balloon layer.

According to a further embodiment, a composite medical balloon is provided. The composite medical balloon comprises balloon including first and second tapered portions having a barrel portion therebetween. A hybrid layer is adhesively attached to the balloon. The hybrid layer comprises a tube covering the barrel portion, a first spirally wrapped ribbon of material covering the first tapered portion, and a second spirally wrapped ribbon of material covering the second tapered portion.

In one embodiment, the first spirally wrapped ribbon of material includes a plurality of overlapping winds, including one wind overlapping an edge of the tube that is adhesively bonded at the overlap. The tube and the balloon may each comprise the same material, such as a polyamide (Nylon). The first and second spirally wrapped ribbons of material comprise a polyether block amide (PEBAX). The tube may be substantially equal in length to a length of the barrel portion of the base balloon layer. The balloon may also include a fiber layer.

The invention further pertains to a method of forming a composite medical balloon. The method comprises providing a balloon having a barrel portion between first and second tapered portions. The method further comprises forming a hybrid layer on the balloon by applying a tube over the barrel portion of the balloon, and applying a spiral wrapping along one or both of the first and second tapered portions.

In one embodiment, the method includes a step of providing an adhesive to the balloon prior to the forming step. The method may further include the step of inflating the balloon prior to the step of providing an adhesive, deflating the balloon prior to the step of applying the tube, and re-inflating the balloon prior to the step of applying the spiral wrapping. The step of applying the spiral wrapping may comprise applying a spirally wrapped ribbon of material to each of the first and second tapered portions of the balloon. The method may further include the step of laminating the balloon, the tube, and the spiral wrapping.

### BRIEF DESCRIPTION OF THE DRAWINGS

The above and further advantages of the disclosure may be better understood by referring to the following description in conjunction with the accompanying drawings in which:
FIG. 1 illustrates a semi-cross section of a medical balloon;
FIG. 2 illustrates an inflated balloon base layer;
FIG. 3 illustrates a balloon-shaped mandrel;
FIG. 4 illustrates a balloon base layer having an adhesive layer;
FIG. 5 illustrates a first fiber layer;
FIG. 6 illustrates a cross-section of a balloon base layer, adhesive layer and first fiber layer;
FIG. 7 illustrates a cross-section of a balloon base layer, adhesive layer and fiber layers;
FIG. 8 illustrates a cross-section of a balloon base layer, an adhesive layer, a first fiber layer, a second fiber layer, an outer coating layer and a final layer;
FIG. 9 illustrates a fiber-reinforced medical balloon with a longitudinal first fiber layer and a circumferential second fiber layer;
FIG. 10 illustrates a fiber-reinforced medical balloon with a first angled fiber layer and a second longitudinal second fiber layer;
FIG. 11 illustrates a fiber-reinforced medical balloon having a first longitudinal fiber layer and a second angled fiber layer;
FIG. 12 illustrates a fiber-reinforced medical balloon having a longitudinal first fiber layer and an angled second fiber layer;
FIG. 13 illustrates an alternative embodiment of a fiber-reinforced medical balloon;
FIG. 14 illustrates an alternative embodiment of a fiber-reinforced medical balloon;

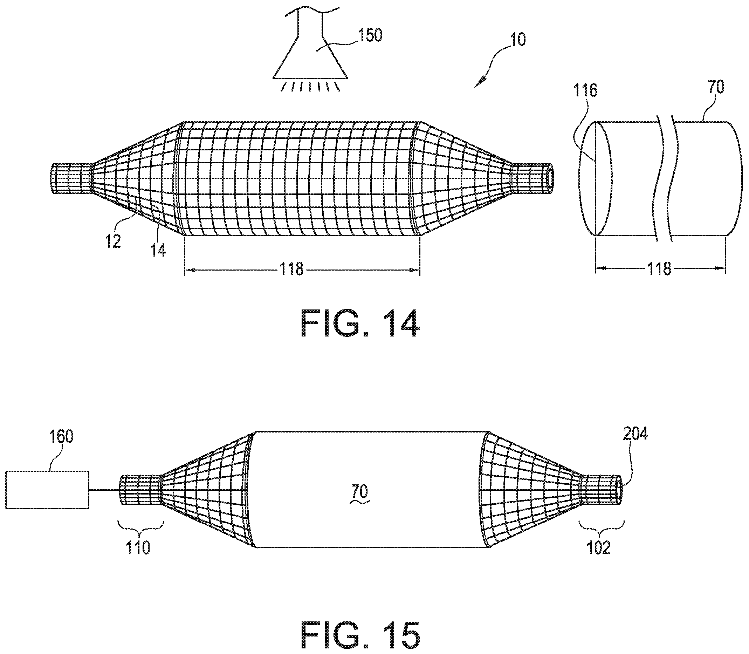

FIGS. 14,15,16,17,17A, and 18 illustrates a manner of providing a fiber-reinforced medical balloon with a hybrid outer layer; and

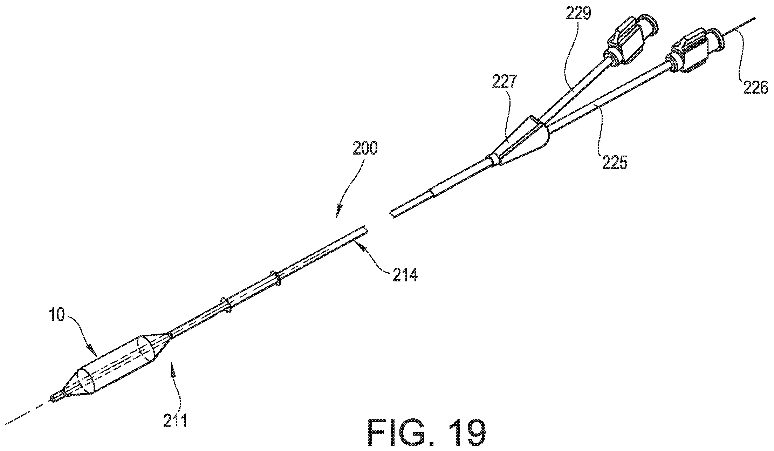

FIGS. 19, 20, 20A, and 21 illustrate a medical balloon with a hybrid outer layer forming part of a catheter.

The dimensions of some of the elements may be exaggerated relative to other elements for clarity or several physical components may be included in one functional block or element. Further, sometimes reference numerals may be repeated among the drawings to indicate corresponding or analogous elements. Moreover, some of the items depicted in the drawings may be combined into a single function.

### DETAILED DESCRIPTION

In the following detailed description, numerous specific details are set forth to provide a thorough understanding of the present invention. In other instances, well-known methods, procedures, components, or structures may not have been described in detail so as not to obscure the present invention.

The principles and operation of systems and methods of the disclosure may be better understood with reference to the drawings and accompanying descriptions. The invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein are for the purpose of description and should not be regarded as limiting.

Certain features of the invention that are, for clarity, described in the context of separate embodiments, may also be provided in combination in a single embodiment. Conversely, various features of the invention that are, for brevity, described in the context of a single embodiment, may also be provided separately or in any suitable subcombination.

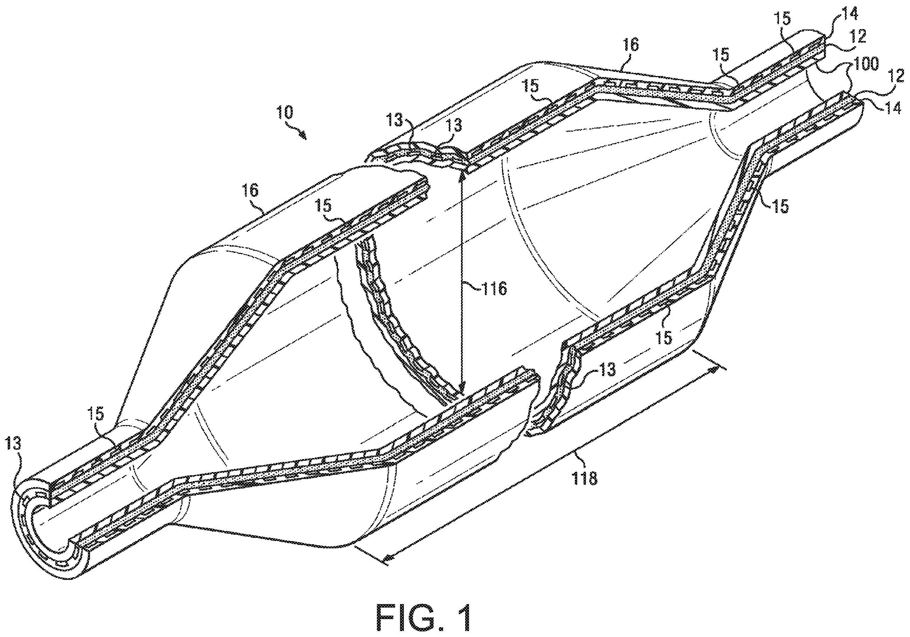

With reference to FIG. 1, a partial cross section of an inflated composite medical balloon 10 is shown. In the illustrated embodiment, and as discussed further in the following description, the balloon 10 may optionally be fiber-reinforced and, as a result, is substantially non-compliant, having limited expansion characteristics. Because the medical balloon 10 is non-compliant, once fully inflated, a diameter 116 of it does not substantially change as the interior pressure increases. As noted further below, the use of a fiber layer is considered entirely optional.

The diameter 116 of an inflated fiber-reinforced medical balloon 10 in accordance with the one embodiment may be about ten millimeters, but may vary depending on the application. The length of an inflated fiber-reinforced medical balloon 10 in accordance with one embodiment may be about eight centimeters. A balloon 10 with a length 118 of 2-200 centimeters or more is possible. The inclination angle of a tapered or cone portion 108 of an inflated fiber-reinforced medical balloon 10 in accordance with the disclosed embodiment may be about twenty degrees. It will be recognized by those having skill in the art that the fiber-reinforced balloon 10 could be made in a wide variety of diameters 116 and lengths and with a variety of inclinations at the tapered or cone portion 108 of the balloon 10, without limitation.

The fiber-reinforced medical balloon 10 may include a base layer 100 formed of a thin polymer material and a first layer 12 of thin inelastic fibers 13. The balloon 10 may include a second layer 14 made up of one or more fibers 15. An outer layer 16 over the fiber layer(s) 12, 14 may be included, as outlined in the following description.

Each fiber 13 is typically fixed relative to other fibers in the first fiber layer 12 and other fibers in the balloon 10. The thin inelastic fibers 13 of the first fiber layer 12 may be characterized by a high tensile strength, which provide superior burst strength. The fiber-reinforced balloon 10 may also resist abrasion, cuts, and punctures. It may be recognized that enhanced structural integrity may result from the fiber reinforcement.

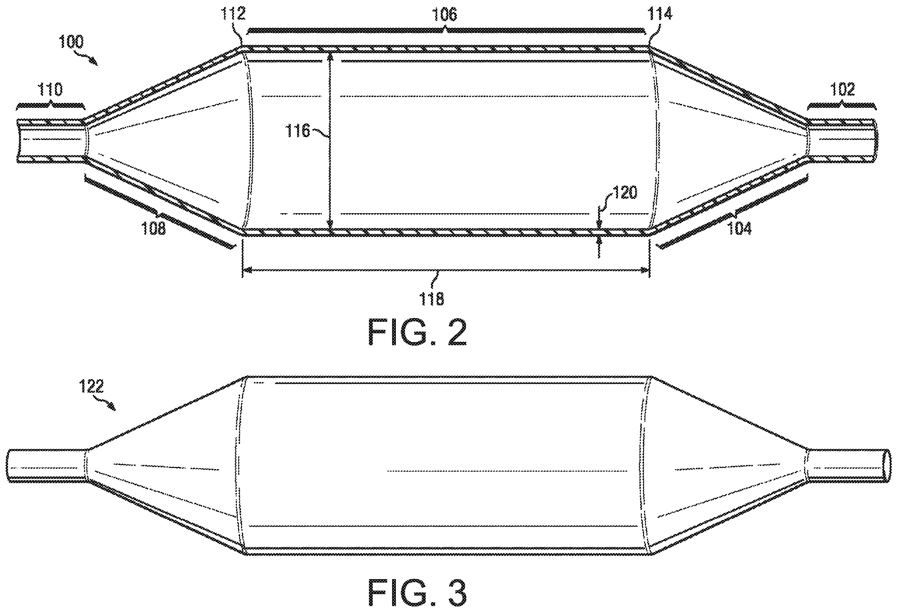

With further reference to FIG. 2, the base layer 100 may be in the shape of a standard medical balloon, or any other suitable shape. The base layer 100 typically includes a first neck portion 102 that may be formed as a narrow cylinder fashioned to attach to a catheter shaft (see FIGS. 19, 20 and 21). A second neck portion 110 may be similarly formed as a narrow tube. The first neck portion 102 is formed adjacent to a first cone or tapered portion 104. The first cone portion 104 expands to meet a barrel portion 106 having a working length 118, marked by a first transition 114. The first cone portion 104 is typically constructed at an angle of about twelve to twenty degrees. The central region or barrel portion 106 meets the second cone or tapered portion 108 at a second transition 112. The second cone portion 108 meets the second neck portion 110.

The base layer 100 is typically formed of a thin film polymeric material, or other suitable materials with high strength relative to film thickness. Polymers and copolymers that can be used for the base layer 100 include the conventional polymers and copolymers used in medical balloon construction, such as, but not limited to, polyethylene, (PET), polycaprolactam, polyesters, polyethers, polyamides, polyurethanes, polyimides, ABS, nylons, copolymers, polyester/polyether block copolymers, ionomer resins, liquid crystal polymers, and rigid rod polymers. The base layer 100 may typically be formed as a blow-molded balloon of a polymer material, such as for example a polyamide, such as nylon.

The base layer 100 may comprise a polymer, which has been cured into the shape of a balloon, may be formed. This base layer 100 of a cured polymer may form the inner polymeric wall of the fiber reinforced balloon. With reference to FIG. 3, a removable mandrel 122 may be used as a base for application of the polymer coating. After the polymer is cured, the mandrel 122 may be removed, such as by physical withdrawal, heat, or other known forms of dissolution.

A removable balloon similar to base layer 100 may be used as the mandrel 122. The mandrel 122 may be made from a variety of materials. The mandrel 122 may be made in the shape of the interior wall of the desired finished balloon. The mandrel 122 may be made of collapsible metal or polymeric bladder, foams, waxes, low-melting metal alloys, and the like. Once the composite balloon is developed and laminated, the mandrel 122 (i.e., base layer 100) may be removed by melting, dissolving, fracturing, compressing, pressurizing, or other suitable removal techniques.

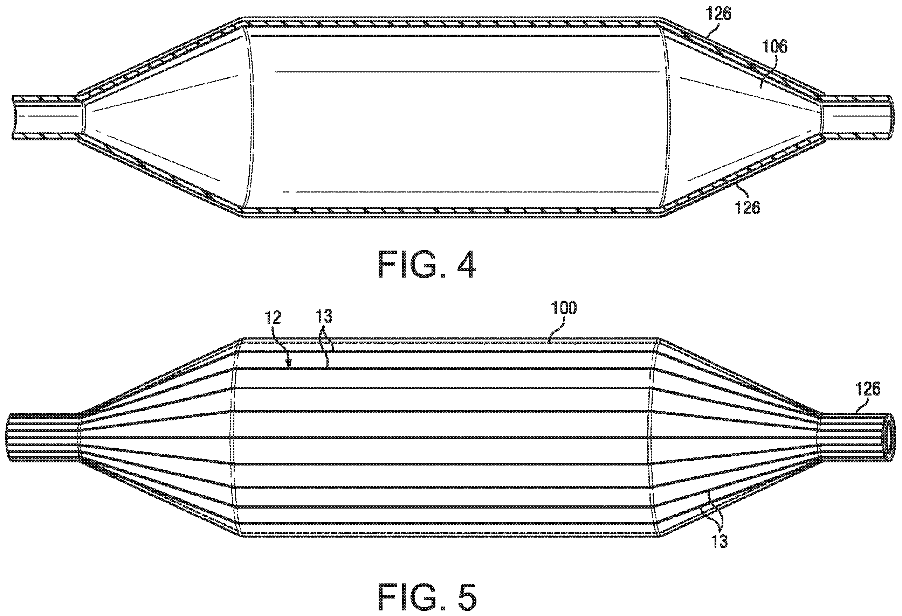

With reference to FIG. 4, it can be understood that a thin coating of an adhesive 126 is applied to the inflated base layer 100 or to the polymer-coated mandrel 122 prior to applying the first layer inelastic fibers 12. The adhesive 126 binds the fibers 13 sufficiently to hold them in position when the fibers 13 are placed on the base layer 100. In accordance with one embodiment, a very thin coat of adhesive 126 is applied to the base layer 100, such as for example I-MP adhesive, which is a known solution of a polyurethane based polymer and methyl ethyl ketone and methylene chloride, but other forms of adhesive could be used.

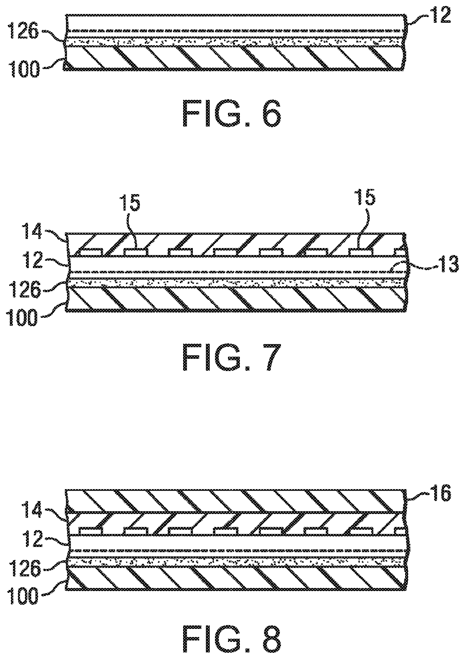

One or more fibers 13 are applied to the base layer 100 to form a first fiber layer 12, as shown in FIGS. 5 and 6, which may be referred to as the "primary wind." The fibers 13 of the first fiber layer 12 may be inelastic fiber, typically made of an inelastic fibrous material. An inelastic fiber is a fiber that has very minimal elasticity or stretch over a given range of pressures. Some fibrous materials are generally classified as inelastic although the all fibrous material may have a detectable, but minimal, elasticity or stretch at a given pressure. The fibers 13 of the first fiber layer 12 may be high-strength fibers, typically made of a high-strength fibrous material. Some high strength inelastic fibrous materials may include Kevlar, Vectran, Spectra, Dacron, Dyneema, Terlon (PBT), Zylon (PBO), Polyimide (PIM), other ultrahigh molecular weight polyethylene, aramids, and the like.

In a disclosed embodiment, the fibers 13 of the first fiber layer 12 are ribbon-shaped, where the width of the fiber is larger than the thickness of the fiber. The fibers 13 may be flat so that the fiber has a rectangular cross-section. The fibers 13 used in the initial layer of fibers 12 may all be fibers 13 made of the same material and the same shape. Fibers 13 made from different materials may be used in the initial fiber layer 12. Fibers 13 made in different shapes may be used in the initial fiber layer 12. Ultrahigh Molecular Weight (UHMW) Polyethylene fiber 13, which has been flattened on a roll mill may be used to form the first fiber layer 12. To the flattened fiber 13 is applied an adhesive, such as the I-MP adhesive. The fibers 13 may be arranged as 30 longitudinal fibers, each substantially equal in length to the length of the balloon 10.

The fibers 13 of the initial fiber layer 12 may be arranged so that each fiber 13 is substantially parallel to the long axis of the balloon 10. The density of the fibers 13 in the initial fiber layer 12 is determined by the number of fibers 13 or fiber winds per inch and the thickness of the fibers 13. In a disclosed embodiment of the first fiber layer 12 having longitudinally-placed fibers 13, a fiber density of generally about 15 to 30 fibers 13 having a fiber thickness of about 0.0005 to 0.001 inch and placed equidistant from one another provide adequate strength for a standard-sized fiber-reinforced medical balloon 10. Each of the fibers 13 is substantially equal in length to the balloon 10. The first fiber layer 12 may prevent longitudinal extension of the completed fiber-reinforced balloon 10.

In accordance with a disclosed embodiment, a second fiber layer 14 made with one or more high-strength inelastic fibers 15 is positioned along circumference of the balloon 10, as shown in FIG. 7. The circumferentially placed fibers 15 may be transverse or substantially transverse to the longitudinal axis of the balloon 10. The circumferential fibers 15 may prevent or minimize distension of the balloon diameter 116 at pressures between the minimal inflation pressure and the balloon burst pressure.

The fibers 15 of the second fiber layer 14 may be inelastic fiber, typically made of an inelastic fibrous material. An inelastic fiber is a member of a group of fibers that have very minimal elasticity or stretch in a given range of pressures. Some fibrous materials are generally classified as inelastic although the all fibrous material may have a detectable, but minimal elasticity or stretch at a given pressure. The fibers 15 of the second fiber layer 14 may be high-strength fibers, typically made of a high-strength fibrous material. Some high strength inelastic fibrous materials may include Kevlar, Vectran, Spectra, Dacron, Dyneema, Terlon (PBT), Zylon (PBO), Polyimide (PIM), other ultra-high molecular weight polyethylene, aramids, and the like.

In a disclosed embodiment, the fibers 15 of the second fiber layer 14 are ribbon-shaped, where the width of the fiber is larger than the thickness of the fiber. The fibers 15 may be flat so that the fiber has a rectangular cross-section. The fibers 15 used in the second layer of fibers 14 may all be fibers 15 made of the same material and the same shape. Fibers 15 made from different materials may be used in the second fiber layer 14. Fibers 15 made in different shapes may be used in the second fiber layer 14. UHMW polyethylene fiber 15, which has been flattened on a roll mill may be used to form the second fiber layer 14. To the flattened fiber 15 is applied a thin coat of an adhesive, such as the I-MP adhesive. The fibers 15 may be arranged as a second fiber layer 14 may have a fiber density of 54 wraps per inch.

The fibers 15 of the second fiber layer 14 may be perpendicular to or substantially perpendicular to the fibers 13 placed longitudinally to form the first fiber layer 12. This transverse placement of the first fiber layer 12 and the second fiber layer 14 allows for maximum radial stability of the fiber-reinforced balloon 10. The placement of the fiber layers 12 and 14 distributes the force on the balloon surface equally, creating pixelized pressure points of generally equal shape, size, and density.

The fibers 13 of the first fiber layer 12 may be the same as or different from the fiber 15 of the second fiber layer 14. Specifically, the fibers 15 of the second fiber layer 14 may be made of a different material or materials than the fibers 13 of the first layer 12. The fibers 15 of the second layer 14 may be shaped differently from the fibers 13 of the first fiber layer 12. The characteristics of the fibers or combination of fibers used for the first or second fiber layers may be determined from the specific properties required from the resulting fiber-reinforced balloon 10.

With respect to the fiber density of the second fiber layer 14, in accordance with the disclosed embodiment, fiber 15 having a thickness of about 0.0005 to 0.001 inch and arranged in parallel lines with about 50 to 80 wraps per inch provides generally adequate strength. A single fiber 15 may form the second fiber layer 14, with the fiber 15 wound in a generally parallel series of circumferential continuous loops.

With reference to FIG. 8, a cross section of the integral layers of a fiber-reinforced medical balloon 10 is shown. The first fiber layer 12 and the second fiber layer 14 may be coated with an outer layer 16. The outer layer 16 may be, in the disclosed embodiment, a hybrid outer layer, as outlined further in the following description. A composite structure typically including a base layer 100, an adhesive 126, a first fiber layer 12, a second fiber layer 14 and an outer layer 16 forming a composite, non-compliant fiber-reinforced balloon 10 particularly suitable for medical uses. Typically, the fibers 13 and 15 are fixed when the fiber-reinforced balloon 10 is initially deflated, and then subsequently inflated and deflated during use.

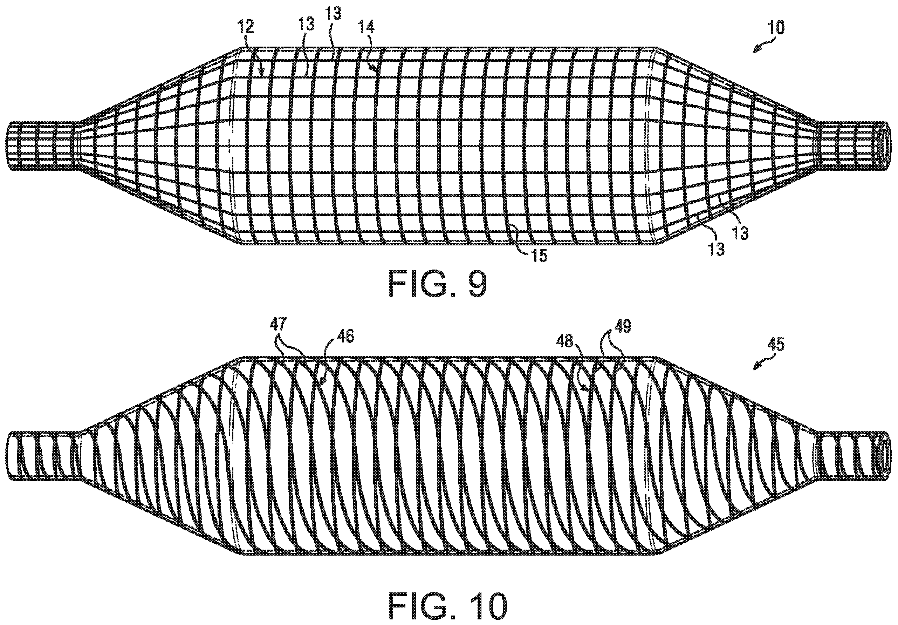

With reference to FIG. 9, a fiber reinforced balloon 10 in accordance with the disclosed embodiment, is shown. In this embodiment, the fibers 13 are parallel to the balloon 10 long axis.

With reference to FIG. 10, a fiber reinforced balloon 45, in accordance with another embodiment is shown. The fiber-reinforced balloon 45 may include a first fiber layer 46 with fibers 47 that lie at an angle to the longitudinal axis of the balloon 45. In this embodiment, neither the fibers 47 of the first fiber layer 46 nor the fibers 49 of the second fiber layer 48 are positioned parallel to the longitudinal axis of the balloon 45. In accordance with one embodiment, the fibers 47 of the first fiber layer 46 may be positioned parallel to a line at a five-degree angle to a line parallel to the longitudinal axis of the balloon 10. In accordance with another embodiment, the fibers 47 of the first fiber layer 46 may be positioned parallel to a line at a twenty-degree angle to a line parallel to the longitudinal axis of the balloon 10.

In accordance with another embodiment, the fibers 47 of the first fiber layer 46 may be positioned parallel to a line at a thirty-degree angle to a line parallel to the longitudinal axis of the balloon 10. In accordance with another embodiment, the fibers 47 of the first fiber layer 46 may be positioned parallel to a line at a forty-five-degree angle to a line parallel to the longitudinal axis of the balloon 10. It will be apparent to those having skill in the art that the fibers 47 may be placed at any appropriate angle.

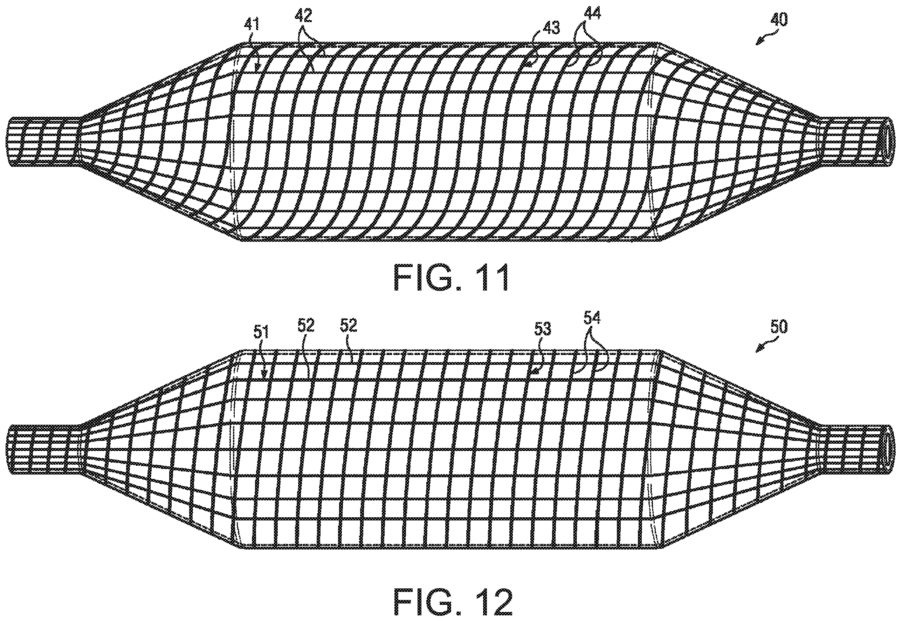

The fibers 49 of the second fiber layer 48 lie parallel to the circumference of the balloon 10. With reference to FIG. 11, a fiber-reinforced balloon 40 in accordance with another embodiment is shown. The fiber reinforced balloon 40 may include a second fiber layer 43 with fibers 44 that lie at an angle to the circumference of the balloon 40. In accordance with one embodiment, the fibers 44 of the second fiber layer 43 may be positioned parallel to a line at a five-degree angle to a line parallel to the circumference of the balloon 10.

The fiber 44 of the second fiber layer 43 may be positioned parallel to a line at a twenty-degree angle to a line parallel to the circumference of the balloon 10. In accordance with one embodiment, the fiber 44 of the second fiber layer 43 may be positioned parallel to a line at a thirty-degree angle to a line parallel to the circumference of the balloon 10. In accordance with one embodiment, the fiber 44 of the second fiber layer 43 may be positioned parallel to a line at a forty-five-degree angle to a line parallel to the circumference of the balloon 10. It will be apparent to those skilled in the art that the fibers 44 may be placed at any appropriate angle.

The fibers 42 of the first fiber layer 41 and the fibers 44 of the second fiber layer 43 are positioned perpendicularly relative to each other. With reference to FIG. 12, a fiber-reinforced balloon 50 in accordance with another embodiment is shown. A fiber-reinforced balloon 50 may include fibers 52 of the first fiber layer 51 and fibers 54 of the second fiber layer 53 positioned relatively at an angle other than a right angle.

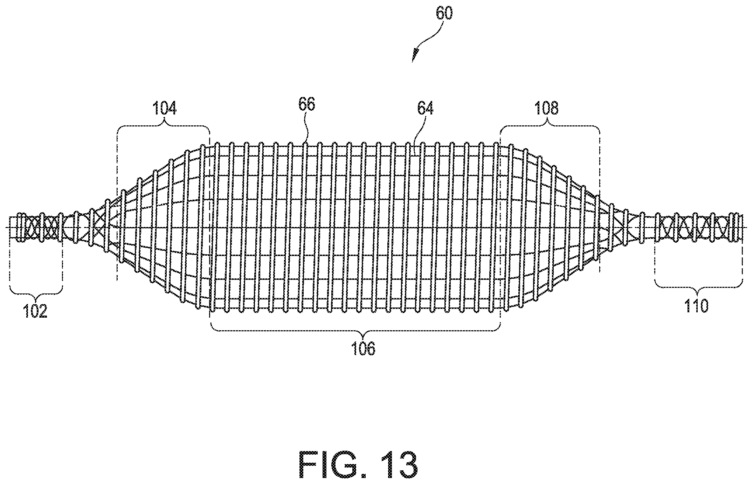

Referring to FIG. 13, a medical balloon 60 may also include a single continuous fiber forming a longitudinal fiber strand 64 and a hoop fiber strand 66. Specifically, the fiber 62 may be wrapped circumferentially around one end portion, such as neck portion 102, and directed longitudinally to the other end portion, or neck portion 110 (possibly at a non-zero angle to the longitudinal axis). The fiber 62 is then wrapped around the other end portion 110, and returned in the opposite longitudinal direction (again, possibly at a non-zero angle). The single continuous fiber 62 can continue to be wound back and forth along the balloon 60 for a desired number of longitudinal passes, and then would circumferentially or helically around the balloon 60 at a desired fiber pitch. A more complete description of the application to a single continuous fiber to a medical balloon 60 may be found in U.S. Patent No. 10,485,949.

Turning to FIG. 14, a manner of forming the outer layer 16 of the balloon 10 is shown (which may apply to any of the embodiments of balloons described herein). The balloon 10 as illustrated is fiber-reinforced, and thus includes one or more fiber layers, and any mandrel is removed, if present. At least the barrel portion 106 of the as-formed balloon 10 is then provided with an adhesive coating, such as I-MP, and at least partially deflated. The application of the adhesive coating to the balloon 10 may be done by spraying using a sprayer 150, as shown, but could also be done using other forms of application, such as dip coating, sputtering, brushing, rolling, or the like. While FIGS. 14 to 17 show one or more fiber layers, however, these are purely optional.

A tube 70 is then positioned over the adhesive coating applied to the base layer 100 and any overlying fiber layers 12,14. The tube 70 may correspond in length 118 to the barrel portion 106 and having an inner diameter 116 may be equal to or greater than the outer diameter of the balloon 10, or may be smaller in diameter and stretched by the base layer 100 on inflation The tube 70 may be fabricated of the same material as the base layer 100 (e.g., a nylon or PET film), and may be formed by extrusion or blow-molding. The tube 70 could also be formed by blow-molding a balloon-shaped structure similar to base layer 100 and cutting off the cone portions 104,108, which results in a tube in a shape and size similar to barrel portion 106.

The balloon 10 with the adhesive coating and the tube 70 in place over the barrel portion 106 is then inflated. This may be achieved by supplying a pressurized fluid (air) from a source 160 through one neck portion 110 and providing a seal 204 at the open end of the other neck portion 102. With the balloon 10 inflated, a different material is applied to at least one of the cone portions 104,108 in the same layer as the tube 70 in the barrel portion 106. Specifically, a ribbon of material 80, such as one comprising a polymer film, such as for example pre-stretched PEBAX film. The material 80 may have a thickness similar to that of the tube 70, and may be spirally wrapped or wound around one or both of the cone portions 104, 108, so as to form a spiral wrapping. The width of the material 80 may be relatively narrow, such that two or more passes or winds are required to cover the entirety of each cone portions 104, 108, but the arrangement will ultimately depend on the relative dimensions of the material 80 and the cone portions 104, 108. Adhesive may also be applied to the material 80 during spiral wrapping, but may alternatively or additionally be applied directly to the cone portions 104,108.

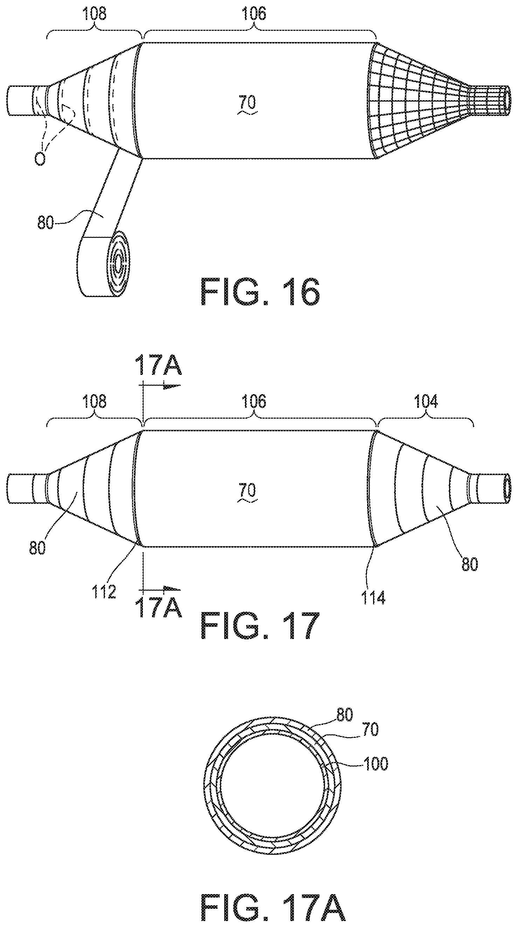

The winding of the material 80 may be done in an overlapping manner. Specifically, each wind or pass of the ribbon of material 80 may at least partially overlap with an adjacent wind (as indicated by phantom lines O indicating overlap). Furthermore, the ribbon of material 80 may overlap with the edges of the tube 70 at the transitions 112, 114 adjacent to the cone portions 104,108, as can be understood by the cross-sectional view of FIG. 17A. Alternatively, if the ribbon of material 80 is applied first, then the edges of the tube 70 could overlap with it instead. Adhesive may be added between overlapped regions to aid in seamless bonding and transition between regions.

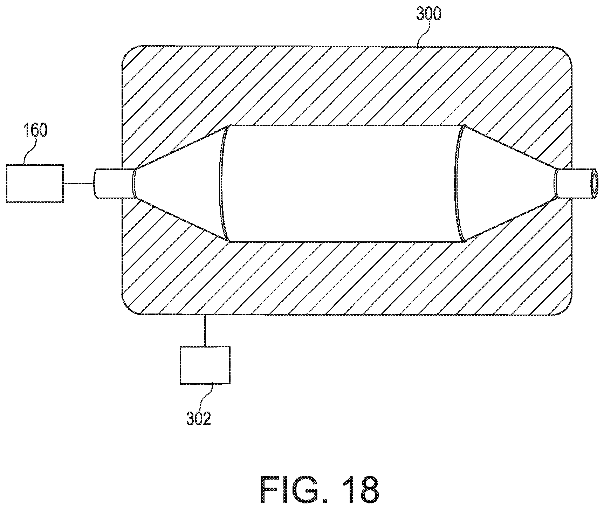

With the tube 70 any spiral wrapping of material 80 in place, the balloon 10 may then be subjected to lamination or consolidation, such as by placing it within a split die 300, as shown in FIG. 18. Heat and pressure may then be applied (note inflation source 202 and heater 203 connected to die 300) to the balloon 10 to activate the previously applied adhesive and allow it to flow along the interface between the tube 70 and material 80 and the underlying base layer 100. Consequently, the tube 70 and the spirally wrapped material 80 becomes fully bonded in place and connected to the underlying layer, such as base layer 100 or any fiber layers 12,14 present.

Advantageously, this creates a balloon 10 having a hybrid outer layer 16 with several distinct advantages. Specifically, the ability to provide a continuous or seamless tube 70 for the barrel portion 106 allows for the selection of a material with superior properties (e.g. porosity, texture, hardless, etc.) in terms of any associated treatment, such as delivery of a treatment agent or drug (e.g., paclitaxel) coated on the barrel portion, or stent retention/release. The use of a different material for covering the cone portions 104, 108 allows for these regions of the composite balloon 10 to be provided with different properties, such as enhanced flexibility or a differential thickness. Using a spiral wrapping further avoids the need for crimping or folding material around the cone portions 104, 108, which can result in bunching of material when the balloon is laminated and/or folded. This bunched material can also unfurl upon balloon expansion during manufacturing and cause process issues, avoided by using spiral wrapping.

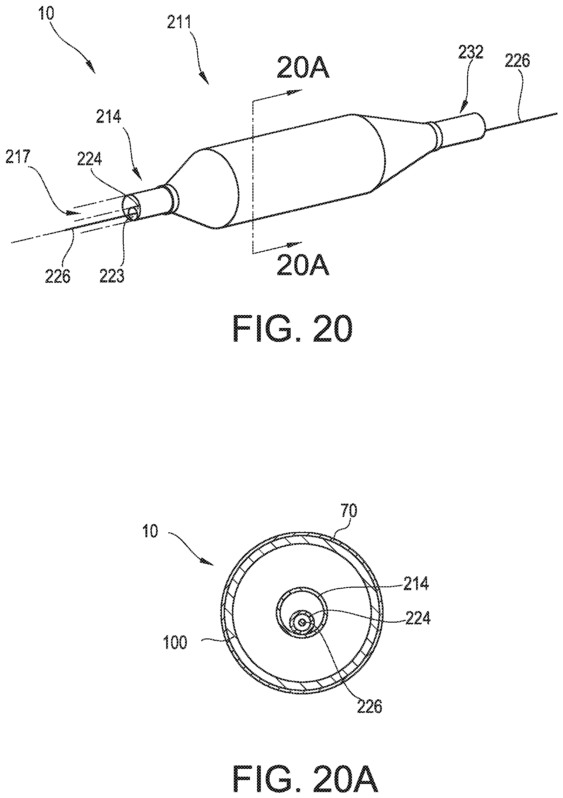

Turning to FIG. 19, it can be understood that the balloon 10 may form part of a catheter 200 having a shaft 214 with a distal end portion 211 to which the balloon 10 is mounted. The balloon 10 is sealed at balloon ends to allow the inflation via one or more inflation lumens 217 extending within catheter shaft 214 and communicating with the interior of the balloon 10. The catheter 200 may also include a guidewire lumen 223 formed by a shaft 224, which may be within the shaft 214 and, more particularly, within the inflation lumen 217. This lumen 223 directs the guidewire 226 through the catheter 200 (see FIG. 20A), and along the distal end portion of which the balloon 10 may be located, including through a tip 232 distal of balloon 10 distal end.

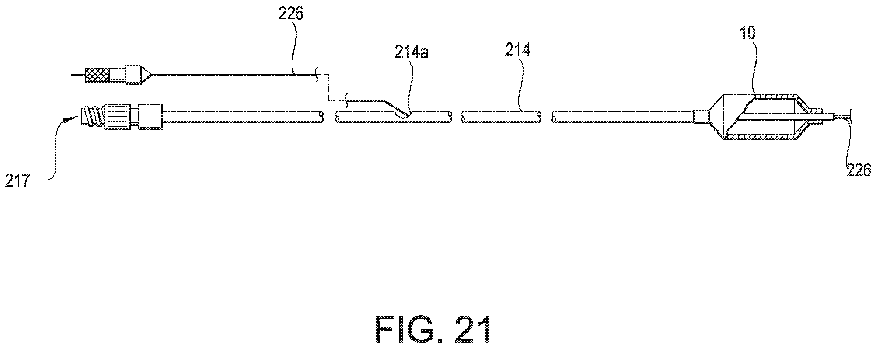

As illustrated in FIG. 20, this guidewire 226 may extend through the proximal end portion of the catheter 200 via a first port 225 of a connector or hub 227 at a proximal end portion 213 of the shaft 214 into the lumen 223 to achieve an "over the wire" (OTW) arrangement, but could also be provided in a "rapid exchange" (RX) configuration (in which the guidewire 226 exits from the shaft 214 at an optional lateral opening 214a (see in FIG. 21) closer to the distal end but proximal of balloon 10) or else is fed through the tip 232 at a passage distally of the balloon 10 ("short" RX; not shown). A second port 229 may also be associated with catheter 200, such as by way of connector 227, for introducing a fluid (e.g., saline, a contrast agent, or both) into the interior compartment of the balloon 10 via the inflation lumen 217.

While the embodiments above and shown and described as a balloon having a fiber layer, it should be understood that this is considered an optional feature. Thus, the balloon 10 could simply comprise the base layer 100 and the hybrid outer layer, as proposed, without the inclusion of fibers. Furthermore, any type of intermediate layer may be provided between the base balloon layer 100 and the hybrid outer later to provide the resulting balloon with desired characteristics, such as a particular degree of compliance or an enhanced resistance to bursting.

The scope of the present invention is defined by the appended claims.

## Claims

1. A composite medical balloon (10), comprising:
a base balloon layer (100) including first and second tapered portions (104, 108) and a barrel portion (106) therebetween; **characterized in that** the composite medical balloon (10) further comprises
a hybrid layer over the base balloon layer, the hybrid layer comprising a tube (70) covering the barrel portion and a spiral wrapping (80) covering at least one of the first and second tapered portions.

2. The composite medical balloon of claim 1, wherein the tube comprises an extruded or blow-molded material.

3. The composite medical balloon of any of claims 1-2,
wherein the tube comprises a polyamide; and/or
wherein the base balloon layer comprises a polyamide.

4. The composite medical balloon of any of claims 1-3, wherein the spiral wrapping comprises a spirally wound ribbon of material extending from one end of the first or second tapered portion to another end of the corresponding tapered portion.

5. The composite medical balloon of any of claims 1-4, wherein the spiral wrapping overlaps with the tube at a transition from the first or second tapered portion to the barrel portion.

6. The composite medical balloon of any of claims 1-5, wherein the spiral wrapping comprises a polyether block amide.

7. The comprise medical balloon of any of claims 1-6, further including a spiral wrapping covering the other of the first or second tapered portions.

8. The composite medical balloon of any of claims 1-7, wherein the tube is substantially equal in length to a length of the barrel portion of the base balloon layer.

9. The composite medical balloon of any of claims 1-8, further including one or more fiber layers (12, 14) between the base balloon layer and the hybrid layer.

10. The composite medical balloon according to claim 1,
wherein the hybrid layer is adhesively attached to the balloon and comprises a first spirally wrapped ribbon of material (80) covering the first tapered portion, and a second spirally wrapped ribbon (80) of material covering the second tapered portion.

11. The composite medical balloon of claim 10, wherein the first spirally wrapped ribbon of material includes a plurality of overlapping winds, including one wind overlapping an edge of the tube.

12. The composite medical balloon of any of claims 10-11,
wherein the tube comprises a polyamide; and/or
wherein the balloon comprises a polyamide; and/or
wherein the first and/or second spirally wrapped ribbons of material comprise a polyether block amide; and/or
wherein the tube is substantially equal in length to a length of the barrel portion of the balloon; and/or
further including a fiber layer (12, 14) between the balloon and the hybrid layer.

13. A method of forming a composite medical balloon (10), comprising:
providing a balloon having a barrel portion (106) between first and second tapered portions (104, 108);
forming a hybrid layer on the balloon by applying a tube (70) over the barrel portion of the balloon, and applying a spiral wrapping (80) along one or both of the first and second tapered portions.

14. The method of claim 13,
further including a step of providing an adhesive (126) to the balloon prior to the forming step; and/or
further including the step of inflating the balloon prior to the step of providing an adhesive, deflating the balloon prior to the step of applying the tube, and re-inflating the balloon prior to the step of applying the spiral wrapping; and/or
wherein the step of applying the spiral wrapping comprises applying a spirally wrapped ribbon of material (80) to each of the first and/or second tapered portions of the balloon; and/or
further including the step of bonding the balloon, the tube, and the spiral wrapping through the application of heat and pressure.

15. The method of any of claims 13-14, further including the step of applying one or more fiber layers (12, 14) to the balloon.

## Patentansprüche

1. Medizinischer Verbundballon (10), umfassend:
eine Grundballonschicht (100), einschließlich erster und zweiter sich verjüngender Abschnitte (104, 108) und einem dazwischen angeordneten Zylinderabschnitt (106); **dadurch gekennzeichnet, dass** der medizinische Verbundballon (10) weiter Folgendes umfasst:
eine Hybridschicht über der Grundballonschicht, wobei die Hybridschicht einen den Zylinderabschnitt abdeckenden Schlauch (70) und eine mindestens einen der ersten und zweiten sich verjüngenden Abschnitte abdeckende Spiralumwicklung (80) umfasst.

2. Medizinischer Verbundballon nach Anspruch 1, wobei der Schlauch ein extrudiertes oder blasgeformtes Material umfasst.

3. Medizinischer Verbundballon nach einem der Ansprüche 1-2, wobei der Schlauch ein Polyamid umfasst; und/oder
wobei die Grundballonschicht ein Polyamid umfasst.

4. Medizinischer Verbundballon nach einem der Ansprüche 1-3, wobei die Spiralumwicklung ein spiralförmig gewickeltes Band aus Material umfasst, das sich von einem Ende des ersten oder zweiten sich verjüngenden Abschnitts zu einem anderen Ende des entsprechenden sich verjüngenden Abschnitts erstreckt.

5. Medizinischer Verbundballon nach einem der Ansprüche 1-4, wobei die Spiralumwicklung den Schlauch an einem Übergangsbereich vom ersten oder zweiten sich verjüngenden Abschnitt zum Zylinderabschnitt überlappt.

6. Medizinischer Verbundballon nach einem der Ansprüche 1-5, wobei die Spiralumwicklung ein Polyetherblockamid umfasst.

7. Medizinischer Verbundballon nach einem der Ansprüche 1-6, weiter einschließend eine Spiralumwicklung, die den anderen der ersten oder zweiten sich verjüngenden Abschnitte abdeckt.

8. Medizinischer Verbundballon nach einem der Ansprüche 1-7, wobei der Schlauch in der Länge im Wesentlichen gleich einer Länge des Zylinderabschnitts der Grundballonschicht ist.

9. Medizinischer Verbundballon nach einem der Ansprüche 1-8, weiter einschließend eine oder mehrere Faserschichten (12, 14) zwischen der Grundballonschicht und der Hybridschicht.

10. Medizinischer Verbundballon nach Anspruch 1,
wobei die Hybridschicht mittels Klebstoff an dem Ballon befestigt ist und ein erstes spiralförmig gewickeltes Band aus Material (80), das den ersten sich verjüngenden Abschnitt abdeckt, und ein zweites spiralförmig gewickeltes Band aus Material (80), das den zweiten sich verjüngenden Abschnitt abdeckt, umfasst.

11. Medizinischer Verbundballon nach Anspruch 10, wobei das erste spiralförmig gewickelte Band aus Material eine Vielzahl überlappender Windungen, einschließlich einer Windung, die einen Rand des Schlauchs überlappt, einschließt.

12. Medizinischer Verbundballon nach einem der Ansprüche 10-11,
wobei der Schlauch ein Polyamid umfasst; und/oder
wobei der Ballon ein Polyamid umfasst; und/oder
wobei das erste und/oder zweite spiralförmig gewickelte Band aus Material ein Polyetherblockamid umfasst; und/oder
wobei der Schlauch in der Länge im Wesentlichen gleich einer Länge des Zylinderabschnitts des Ballons ist; und/oder
weiter einschließend eine Faserschicht (12, 14) zwischen dem Ballon und der Hybridschicht.

13. Verfahren zum Formen eines medizinischen Verbundballons (10), umfassend:
Bereitstellen eines Ballons, der einen Zylinderabschnitt (106) zwischen ersten und zweiten sich verjüngenden Abschnitten (104, 108) aufweist;
Ausbilden einer Hybridschicht auf dem Ballon durch Anbringen eines Schlauchs (70) über dem Zylinderabschnitt des Ballons, und Anbringen einer Spiralumwicklung (80) entlang eines oder beider der ersten und zweiten sich verjüngenden Abschnitte.

14. Verfahren nach Anspruch 13,
weiter einschließend einen Schritt des Bereitstellens eines Klebstoffs (126) an dem Ballon vor dem Formschritt; und/oder
weiter einschließend den Schritt des Aufblasens des Ballons vor dem Schritt des Bereitstellens eines Klebstoffs, des Entleerens des Ballons vor dem Schritt des Anbringens des Schlauchs, und des erneuten Aufblasens des Ballons vor dem Schritt des Anbringens der Spiralumwicklung; und/oder
wobei der Schritt des Anbringens der Spiralumwicklung das Anbringen eines spiralförmig gewickelten Bandes aus Material (80) an jedem der ersten und/oder zweiten sich verjüngenden Abschnitte des Ballons umfasst; und/oder
weiter einschließend den Schritt, den Ballon, den Schlauch und die Spiralumwicklung durch Aufbringen von Wärme und Druck miteinander zu verbinden.

15. Verfahren nach einem der Ansprüche 13-14, weiter einschließend den Schritt des Anbringens einer oder mehrerer Faserschichten (12, 14) an dem Ballon.

## Revendications

1. Ballon médical composite (10), comprenant :
une couche de ballon de base (100) incluant des première et seconde portions coniques (104, 108) et une portion formant cylindre (106) entre celles-ci ; **caractérisé en ce que** le ballon médical composite (10) comprend en outre
une couche hybride sur la couche de ballon de base, la couche hybride comprenant un tube (70) recouvrant la portion formant cylindre et un enroulement en spirale (80) recouvrant au moins l'une des première et seconde portions coniques.

2. Ballon médical composite selon la revendication 1, dans lequel le tube comprend un matériau extrudé ou moulé par soufflage.

3. Ballon médical composite selon l'une quelconque des revendications 1 à 2,
dans lequel le tube comprend un polyamide ; et/ou
dans lequel la couche de ballon de base comprend un polyamide.

4. Ballon médical composite selon l'une quelconque des revendications 1 à 3, dans lequel l'enroulement en spirale comprend un ruban de matériau enroulé en spirale s'étendant d'une extrémité de la première ou de la seconde portion conique jusqu'à une autre extrémité de la portion conique correspondante.

5. Ballon médical composite selon l'une quelconque des revendications 1 à 4, dans lequel l'enroulement en spirale chevauche le tube au niveau d'une transition de la première ou de la seconde portion conique vers la portion formant cylindre.

6. Ballon médical composite selon l'une quelconque des revendications 1 à 5, dans lequel l'enroulement en spirale comprend un polyéther bloc amide.

7. Ballon médical composite selon l'une quelconque des revendications 1 à 6, incluant en outre un enroulement en spirale recouvrant l'autre de la première ou de la seconde portion conique.

8. Ballon médical composite selon l'une quelconque des revendications 1 à 7, dans lequel le tube a une longueur sensiblement égale à une longueur de la portion formant cylindre de la couche de ballon de base.

9. Ballon médical composite selon l'une quelconque des revendications 1 à 8, incluant en outre une ou plusieurs couches de fibres (12, 14) entre la couche de ballon de base et la couche hybride.

10. Ballon médical composite selon la revendication 1,
dans lequel la couche hybride est fixée au moyen d'un adhésif au ballon et comprend un premier ruban (80) de matériau enroulé en spirale recouvrant la première portion conique, et un second ruban (80) de matériau enroulé en spirale recouvrant la seconde portion conique.

11. Ballon médical composite selon la revendication 10, dans lequel le premier ruban de matériau enroulé en spirale inclut une pluralité de spires se chevauchant, incluant une spire chevauchant un bord du tube.

12. Ballon médical composite selon l'une quelconque des revendications 10 à 11,
dans lequel le tube comprend un polyamide ; et/ou
dans lequel le ballon comprend un polyamide ; et/ou
dans lequel le premier et/ou le second ruban de matériau enroulé en spirale comprend un polyéther bloc amide ; et/ou
dans lequel le tube a une longueur sensiblement égale à une longueur de la portion formant cylindre du ballon ; et/ou
incluant en outre une couche de fibres (12, 14) entre le ballon et la couche hybride.

13. Procédé de formage d'un ballon médical composite (10), comprenant :
fournir un ballon présentant une portion formant cylindre (106) entre les première et seconde portions coniques (104, 108) ;
former une couche hybride sur le ballon en appliquant un tube (70) sur la portion formant cylindre du ballon, et en appliquant un enroulement en spirale (80) le long de l'une ou des deux des première et seconde portions coniques.

14. Procédé selon la revendication 13,
incluant en outre une étape consistant à fournir un adhésif (126) au ballon avant l'étape de formage ; et/ou
incluant en outre l'étape consistant à gonfler le ballon avant l'étape consistant à fournir un adhésif, à dégonfler le ballon avant l'étape consistant à appliquer le tube, et à regonfler le ballon avant l'étape consistant à appliquer l'enroulement en spirale ; et/ou
dans lequel l'étape consistant à appliquer l'enroulement en spirale comprend l'étape consistant à appliquer un ruban (80) de matériau enroulé en spirale sur chacune des première et/ou seconde portions coniques du ballon ; et/ou
incluant en outre l'étape consistant à lier le ballon, le tube, et l'enroulement en spirale par application de chaleur et de pression.

15. Procédé selon l'une quelconque des revendications 13 à 14, incluant en outre l'étape consistant à appliquer une ou plusieurs couches de fibres (12, 14) sur le ballon.
